# EUROPÄISCHE PATENTANMELDUNG

(11) **EP 1 792 895 A1**
(43) Veröffentlichungstag der Anmeldung: **06.06.2007**
(21) Anmeldenummer: 06023121.4
(22) Anmeldetag: 07.11.2006
(51) Int. Cl.: C07C 263/10, C07C 263/20, C07C 265/14, C08G 18/76

(54) **Verfahren zur Herstellung von 4,4'-Diphenylmethandiisocyanat**

(30) Priorität: 19.11.2005 DE 102005055189
(71) Anmelder: Bayer MaterialScience AG, 51368 Leverkusen (DE)
(72) Erfinder: Keggenhoff, Berthold, Dr., 47839 Krefeld (DE); Bolton, Jeffrey, Dr., 40489 Düsseldorf (DE)

(57) **Zusammenfassung**

Die Erfindung betrifft ein Verfahren zur Herstellung von 4,4'-Diphenylmethandiisocyanat (4,4'-MDI) durch säurekatalysierte Kondensation von Anilin mit Formaldehyd, Umsetzung der erhaltenen Gemische von Di- und Polyaminen mit Phosgen zu dem entsprechenden MDI-Isomeren- und Homologengemisch (Di- und Polyisocyanate der Diphenylmethanreihe) und anschließende destillative Auftrennung des Gemischs zu 4,4'-MDI und polymerem MDI.

## Beschreibung

Die Erfindung betrifft ein Verfahren zur Herstellung von 4,4'-Diphenylmethandiisocyanat (4,4'-MDI) durch säurekatalysierte Kondensation von Anilin mit Formaldehyd, Umsetzung der erhaltenen Gemische von Di- und Polyaminen mit Phosgen zu dem entsprechenden MDI-Isomeren- und Homologengemisch (Di- und Polyisocyanate der Diphenylmethanreihe) und anschließende destillative Auftrennung des Gemischs zu 4,4'-MDI und polymerem MDI. Dabei werden die bei der Aufarbeitung des Di- und Polyisocyanatgemischs zu 4,4'-MDI ggf. anfallenden Nebenströme bevorzugt zurück in die Aufarbeitung des Polyisocyanatgemischs geführt und/ oder dem polymeren MDI zugemischt, so dass als Verfahrensprodukte nur technisch reines 4,4'-MDI und handelsübliches polymeres MDI erhalten werden.

Üblicherweise wird die großtechnische Herstellung von 4,4'-MDI mit den Verfahrensstufen säurekatalysierte Kondensation von Anilin mit Formaldehyd, Umsetzung der erhaltenen Polyamingemische mit Phosgen zu dem MDI-Isomeren- und Homologengemisch (Di- und Polyisocyanate der Diphenylmethanreihe) und anschließende destillative Auftrennung des Gemischs zu technisch reinem 4,4'-MDI, polymerem MDI und weiteren Isomerengemischen durchgeführt. Wie z.B. in EP-A-1475367 beschrieben, wird hierzu zunächst das MDI-Isomeren- und Homologengemisch (Di- und Polyisocyanate der Diphenylmethanreihe) hergestellt. Dann wird aus diesem ein Teildestillat an isomeren Diphenylmethandiisocyanaten mit dem Hauptbestandteil 4,4'-MDI abgetrennt, wobei das Sumpfprodukt als polymeres MDI technisch verwendet wird. Anschließend wird das Teildestillat in reines 4,4'-MDI, ein Gemisch von 4,4'-MDI mit 2,4'-MDI und ggf. 2,2'-MDI und sonstige Nebenprodukte getrennt. Wie in WO-A-02/ 070581 beschrieben wird dabei beispielsweise 2,4'-MDI als Zielprodukt erhalten.

Während es also Märkte gibt, in denen 2,4'-MDI-Destillate für spezielle Anwendungen verwendet werden, gibt es andere Märkte und Regionen, wo solche Produkte praktisch unverkäuflich sind, da die speziellen Anwendungsgebiete fehlen. Speziell für die Produktion von 4,4'-MDI ist aber eine Produktionsstätte in unmittelbarer Nähe der Abnehmermärkte von großem Vorteil, da das Produkt bei Umgebungstemperatur fest ist, in flüssigem Zustand aber wegen seiner Neigung zur Dimerenbildung nur begrenzte Zeit lagerfähig ist, so dass bei Transport über weite Entfernung ein aufwendiger Transport in festem Zustand unter dauernder Kühlung erforderlich ist. Wenn dann in einem solchen Markt kein Bedarf für 2,4'-MDI besteht, stellt sich die Aufgabe, ausschließlich die Verfahrensprodukte 4,4'-MDI und polymeres MDI, und zwar in dem vom Markt gewünschten Verhältnis und mit den vom Markt vorgegebenen Qualitätsanforderungen, herzustellen. Gleichzeitig müssen aber die zwangsläufig bei der Kondensation von Anilin und Formaldehyd anfallenden übrigen Isomere, die dann durch Phosgenierung zu den entsprechenden Isomeren 2,2'- und 2,4'-MDI umgesetzt werden, einer sinnvollen Verwendung zugeführt werden.

Die Aufgabe der vorliegenden Erfindung besteht daher darin, ein einfaches Verfahren zur Herstellung von 4,4'-MDI durch säurekatalysierte Kondensation von Anilin mit Formaldehyd, Umsetzung der erhaltenen Polyamingemische mit Phosgen zu dem MDI-Isomeren- und Homologengemisch und anschließende destillative Auftrennung des Gemischs zu 4,4'-MDI und polymerem MDI zur Verfügung zu stellen, bei dem als einzige Verfahrensprodukte technisch reines 4,4'-MDI und polymeres MDI in technisch üblicher Form und in weitem Bereich einstellbarem Verhältnis erhalten werden.

Gegenstand der Erfindung ist ein Verfahren zur Herstellung von 4,4'-Diphenylmethandiisocyanat, bei dem
a) Anilin und Formaldehyd im Molverhältnis 1,7 bis 4 : 1 in Gegenwart eines sauren Katalysators zu Di- und Polyaminen der Diphenylmethanreihe umgesetzt werden, und
b) die Di- und Polyamine mit Phosgen zu den entsprechenden Di- und Polyisocyanaten der Diphenylmethanreihe umgesetzt werden, und ggf. destillativ getrennt werden, wobei ein Gemisch von Di- und Polyisocyanaten enthaltend 44 - 80 Gew.-%, bevorzugt 50 - 75 Gew.-%, besonders bevorzugt 55 - 70 Gew.-% 4,4'-Diphenylmethandiisocyanat, sowie 1 bis 12 Gew.-%, bevorzugt 2 - 10 Gew.-%, besonders bevorzugt 4 - 9 Gew.-% an 2,4'- und / oder 2,2'- Diphenylmethandiisocyanat in der Summe, und 10 bis 55 Gew.-%, bevorzugt 20 bis 48 Gew.-%, besonders bevorzugt 26 - 40 Gew.-% an Tri- und höherfunktionellen Polyisocyanaten, bezogen auf das Gewicht des Gemisches von Di- und Polyisocyanaten, erhalten wird, und
c) das Gemisch von Di- und Polyisocyanaten durch Destillation und / oder durch Kristallisation in genau zwei Fraktionen aufgetrennt wird, wobei die erste Fraktion in Mengen von 5 - 40 Gew.-%, bevorzugt von 10 - 30 Gew. % der Menge des Gemisches der Di- und Polyisocyanate erhalten wird und die zweite Fraktion in Mengen von 60 - 95 Gew.-%, bevorzugt von 70 - 90 Gew. % der Menge des Gemisches der Di- und Polyisocyanate erhalten wird, und wobei die erste Fraktion mindestens 97 Gew.-% an 4,4'-Diphenylmethandiisocyanat sowie maximal 3 Gew. % an 2,4'-Diphenylmethandiisocyanat, bevorzugt mindestens 98 Gew.-% an 4,4'-Diphenylmethandiisocyanat sowie maximal 2 Gew. % an 2,4'-Diphenylmethandiisocyanat, und maximal 0,1 Gew. % an 2,2'-Diphenylmethandiisocyanat, bezogen auf das Gewicht der ersten Fraktion, enthält und die zweite Fraktion 30 - 60 Gew. % an 4,4'-Diphenylmethandiisocyanat, 1 - 12 Gew. % an 2,4'-Diphenylmethandiisocyanat und maximal 2 Gew. % an 2,2'-Diphenylmethandiisocyanat sowie 35 - 65 Gew. % an Tri- und höherfunktionellen Polyisocyanaten, bevorzugt 35 - 55 Gew. % an 4,4'-Diphenylmethandiisocyanat, 2 - 10 Gew. % an 2,4'-Diphenylmethandiisocyanat und maximal 1 Gew. % an 2,2'-Diphenylmethandiisocyanat sowie 40 - 60 Gew. % an Tri- und höherfunktionellen Polyisocyanaten , bezogen auf das Gewicht der zweiten Fraktion, enthält. Bevorzugt beträgt die Viskosität dieser zweiten Fraktion, gemessen bei 25°C, 40 bis 2000 mPas, besonders bevorzugt 100 bis 800 mPas, gemessen nach DIN 53015/ISO 12058.

Dabei werden in einer bevorzugten Ausführungsform des Verfahrens in Schritt c) ggf. anfallende Nebenströme der Destillation und / oder der Kristallisation der zweiten Fraktion zugeschlagen bzw. derart im Prozess der Aufarbeitung des Gemischs von Di- und Polyisocyanaten rückgeführt, dass sie letztlich in dieser zweiten Fraktion verbleiben.

Die technische Herstellung von Di- und Polyaminen der Diphenylmethanreihe (MDA) in Schritt a) erfolgt im Allgemeinen in zwei Stufen, wobei in der ersten Stufe zunächst eine säurekatalysierte Kondensation von Anilin mit Formaldehyd zur Erzeugung des entsprechenden MDA-Gemisches erfolgt. Dabei wird als Säurekatalysator üblicherweise eine starke Mineralsäure wie wässrige Salzsäure eingesetzt. Verfahren zur Herstellung von MDA durch säurekatalysierte Anilin-Formaldehydkondensation sind in großer Zahl bekannt geworden (z.B. WO-A-99/ 40059, WO-A-99/54289).

Durch Auswahl der Mengenverhältnisse von Anilin, Formaldehyd und Mineralsäure sowie der Temperatur- und Verweilzeitbedingungen können die Anteile an 4,4'-Diphenylmethandiamin sowie dessen Isomeren und Homologen gesteuert werden. Dabei hat sich gezeigt, dass durch Variation des Molverhältnisses von Anilin und Formalin im Bereich 1,7 : 1 bis 4 : 1 bei Einsatz von 0,08 bis 0,3 Mol Mineralsäure pro Mol Anilin das Verhältnis der Endprodukte 4,4'-MDI zu polymerem MDI im Bereich 5 : 95 Gew. Teile bis 40 : 60 Gew. Teile variiert werden kann, ohne das polymere MDI in seinen anwendungstechnischen Eigenschaften spürbar zu verändern.

Die Kondensation kann technisch sowohl kontinuierlich als auch diskontinuierlich durchgeführt werden.

In einer zweiten Stufe wird die erhaltene Reaktionslösung, ggf. nach Neutralisation und Phasentrennung , destillativ von Wasser und Anilin zum MDA aufgearbeitet.

In Schritt b) werden die Di- und Polyamine der Diphenylmethanreihe dann durch Reaktion mit Phosgen in die entsprechenden Di- und Polyisocyanate der Diphenylmethanreihe umgesetzt. Diese Umsetzung erfolgt üblicherweise in einem inerten Lösungsmittel wie Chlorbenzol, Dichlorbenzol oder Toluol, wobei Lösungen von Amin und Phosgen vermischt und anschließend durch Erhitzen ausreagiert werden. Anschließend wird der mitentstandene Chlorwasserstoff sowie überschüssiges Phosgen üblicherweise abgetrennt und das verwendete Lösungsmittel, in der Regel stufenweise, abgetrennt. Nach teilweiser oder bevorzugt vollständiger Abtrennung des Lösungsmittels werden als Sumpfprodukt die Di- und Polyisocyanate der Diphenylmethanreihe erhalten. Auch Verfahren zur Herstellung der Di- und Polyisocyanate der Diphenylmethanreihe durch Umsetzung von Di- und Polyaminen der Diphenylmethanreihe mit Phosgen sind in großer Zahl bekannt (z.B. WO-A-99/ 54289). In Schritt b) wird so, ggf. nach destillativer Aufarbeitung, ein Gemisch von Di- und Polyisocyanaten enthaltend 44 - 80 Gew.-%, bevorzugt 50 - 75 Gew.-%, besonders bevorzugt 55 - 70 Gew.-% 4,4'-Diphenylmethandiisocyanat, sowie 1 bis 12 Gew.-%, bevorzugt 2 - 10 Gew.-%, besonders bevorzugt 4 - 9 Gew.-% an 2,4'- und / oder 2,2'- Diphenylmethandiisocyanat in der Summe, und 10 bis 55 Gew.-%, bevorzugt 20 bis 48 Gew.-%, besonders bevorzugt 26 - 40 Gew.-% an Tri- und höherfunktionellen Polyisocyanaten, bezogen auf das Gewicht des Gemisches von Di- und Polyisocyanaten, erhalten.

In Schritt c) werden aus den Gemischen von Di- und Polyisocyanaten enthaltend 44 - 80 Gew.-% 4,4'-Diphenylmethandiisocyanat, sowie 1 bis 12 Gew.-% an 2,4'- und / oder 2,2'- Diphenylmethandiisocyanat in der Summe, und 10 bis 55 Gew.-% an Tri- und höherfunktionellen Polyisocyanaten, bezogen auf das Gewicht des Gemisches von Di- und Polyisocyanaten dann durch Destillation und / oder Kristallisation (z.B. gemäß DE-A-1938384, DE-A-2631168, EP-A-79516, EP-A-1475367) zwei Fraktionen abgetrennt, wobei die erste Fraktion mindestens 97 Gew.-% an 4,4'-Diphenylmethandiisocyanat sowie maximal 3 Gew. % an 2,4'-Diphenylmethandiisocyanat, bevorzugt mindestens 98 Gew.-% an 4,4'-Diphenylmethandiisocyanat sowie maximal 2 Gew. % an 2,4'-Diphenylmethandiisocyanat und maximal 0,1 Gew. % an 2,2'-Diphenylmethandiisocyanat, bezogen auf das Gewicht der ersten Fraktion, enthält und die zweite Fraktion 30 - 60 Gew. % an 4,4'-Diphenylmethandiisocyanat, 1 - 12 Gew. % an 2,4'-Diphenylmethandiisocyanat und maximal 2 Gew. % an 2,2'-Diphenylmethandiisocyanat sowie 35 - 65 Gew. % an Tri- und höherfunktionellen Polyisocyanaten, bevorzugt 35 - 55 Gew. % an 4,4'-Diphenylmethandiisocyanat, 2 - 10 Gew.% an 2,4'-Diphenylmethandiisocyanat und maximal 1 Gew. % an 2,2'-Diphenylmethandiisocyanat sowie 40 - 60 Gew. % an Tri- und höherfunktionellen Polyisocyanaten, bezogen auf das Gewicht der zweiten Fraktion, enthält. Bevorzugt beträgt die Viskosität dieser zweiten Fraktion, gemessen bei 25°C, 40 bis 2000 mPas, besonders bevorzugt 100 bis 800 mPas, gemessen nach DIN 53015/ ISO 12058.

Die Destillation und / oder Kristallisation kann dabei mehrere Destillations- und / oder Kristallisationsschritte enthalten. Bei dieser Destillation und / oder Kristallisation werden ausschließlich zwei Produktströme entnommen und insbesondere auf die Entnahme von im Wesentlichen monomeren (d.h. Diisocyanat enthaltenden), in größeren Mengen 2,4'-MDI enthaltenden Strömen verzichtet. Denn solche im Wesentlichen monomeren (d.h. Diisocyanat enthaltende) und in größeren Mengen 2,4'-MDI enthaltenden Ströme sind in manchen Regionen nur schwer vermarktbar.

Das Verfahren wird bevorzugt wie folgt durchgeführt: Als erster Verfahrensschritt erfolgt bevorzugt eine partielle Abtrennung der Diisocyanate (monomeres MDI) durch Destillation und / oder Kristallisation, wobei ein Sumpfprodukt bzw. Sumpfstrom enthaltend Di- und Polyisocyanate und eine Fraktion enthaltend mindestens 95 Gew.-% Diisocyanate, bezogen auf das Gewicht der Fraktion, erhalten wird (Polymer- / Monomer - Abtrennung). Dabei kann die abgetrennte Fraktion enthaltend mindestens 95 Gew.-% Diisocyanate (monomeres MDI) bereits die Qualitätsanforderungen für technisch reines 4,4'-MDI, d.h. mindestens 97 Gew.-% an 4,4'-Diphenylmethandiisocyanat sowie maximal 3 Gew. % an 2,4'-Diphenylmethandiisocyanat und maximal 0,5 Gew. % andere Bestandteile, bezogen auf das Gewicht der Fraktion, erfüllen und wird in diesem Fall bevorzugt direkt als erste Fraktion entnommen. Wenn die abgetrennte Fraktion enthaltend mindestens 95 Gew.-% Diisocyanate (monomeres MDI) jedoch nicht die Qualitätsanforderungen für technisch reines 4,4'-MDI erfüllt, wird die Fraktion enthaltend mindestens 95 Gew.-% Diisocyanate (monomeres MDI) bevorzugt in weiteren Destillations- und / oder Kristallisationsschritten von den 2,2'- und 2,4' -Isomeren sowie anderen Verunreinigungen zumindest teilweise befreit, wobei 2,2'-MDI, 2,4'-MDI und / oder andere Verunreinigungen enthaltende Nebenströme anfallen. Als ein Verfahrensprodukt wird jedoch in jedem Fall als erste Fraktion technisch reines 4, 4'-MDI enthaltend mindestens 97 Gew.-% an 4,4'-Diphenylmethandiisocyanat sowie maximal 3 Gew.-% an 2,4'-Diphenylmethandiisocyanat, bezogen auf das Gewicht der Fraktion, erhalten.

Das Sumpfprodukt bzw. der Sumpfstrom enthaltend Di- und Polyisocyanate wird mit den ggf. bei der Destillation und / oder Kristallisation anfallenden Nebenströmen vereinigt, und zu kommerziell üblichen polymerem MDI in unterschiedlichen Viskositätseinstellungen im Bereich 40 bis 2000 mPas, üblicherweise 100 bis 800 mPas, jeweils gemessen nach DIN 53015/ ISO 12058 bei 25°C, verarbeitet. Je nach Qualität der einzelnen Ströme kann dies im einfachsten Fall durch einfaches Abmischen erfolgen und die zweite Fraktion auf diese Weise erhalten werden. Es kann aber auch vorteilhaft sein, die Mischung nochmals zu erhitzen und einer erneuten Destillation, vorzugsweise einer Flashdestillation zu unterwerfen oder die Nebenströme an eine andere Stelle im Prozess der Aufarbeitung des Gemischs von Di- und Polyisocyanaten rückzuführen, wobei in allen Fällen als Sumpfprodukt die zweite Fraktion enthaltend 30 - 60 Gew. % an 4,4'-Diphenylmethandiisocyanat, 1 - 12 Gew. % an 2,4'-Diphenylmethandiisocyanat und maximal 2 Gew. % an 2,2'-Diphenylmethandiisocyanat sowie 35 - 65 Gew. % an Tri- und höherfunktionellen Polyisocyanaten, bevorzugt 35 - 55 Gew. % an 4,4'-Diphenylmethandiisocyanat, 2 - 10 Gew. % an 2,4'-Diphenylmethandiisocyanat und maximal 1 Gew. % an 2,2'-Diphenylmethandiisocyanat sowie 40 - 60 Gew. % an Tri- und höherfunktionellen Polyisocyanaten , bezogen auf das Gewicht der zweiten Fraktion, erhalten wird.

Das Destillat kann wieder der in Schritt c) bevorzugt in der Polymer- / Monomer- Abtrennung entnommenen Fraktion enthaltend mindestens 95 Gew.-% Diisocyanate zugemischt werden. Schließlich ist es auch möglich, alle Sumpf- und Destillatströme mit Ausnahme der ersten Fraktion dem in Schritt b) erhaltenen Gemisch an Di- und Polyisocyanaten der Diphenylmethanreihe wieder zuzumischen und dann im Schritt c) im ersten Verfahrensschritt als Sumpfprodukt kommerziell übliches polymeres MDI als zweite Fraktion, bevorzugt mit der gewünschten Viskosität im Bereich 40 bis 2000 mPas, üblicherweise 100 bis 800 mPas, zu erhalten. Natürlich ist es grundsätzlich auch möglich, einzelne Nebenströme der Destillation und / oder Kristallisation der Fraktion enthaltend mindestens 95 Gew.-% Diisocyanate bzw. der ersten Fraktion zuzuschlagen sofern sicher gestellt ist, dass die erste Fraktion die Qualitätsanforderungen für technisch reines 4,4'-MDI, d.h. mindestens 97 Gew.-% an 4,4'-Diphenylmethandiisocyanat sowie maximal 3 Gew. % an 2,4'-Diphenylmethandiisocyanat, bezogen auf das Gewicht der Fraktion, erfüllt und insgesamt genau zwei Fraktionen, nämlich die erste Fraktion und die zweite Fraktion, erhalten und entnommen werden.

Das erfindungsgemäße Verfahren wird möglich durch die Erkenntnis, dass das Verhältnis der Anteile der verschiedenen Diisocyanat-Isomere (2,2'-, 2,4'-, 4,4'-MDI) untereinander und zu der Menge der Tri- und höherfunktionellen Polyisocyanate in der zweiten Fraktion (polymeres MDI) innerhalb bestimmter Grenzen variiert werden kann, ohne die anwendungstechnischen Eigenschaft des polymeren MDI in größerem Umfang zu verändern. Daher resultiert die angegebene Spezifikation für die zweite Fraktion von 30 - 60 Gew. % an 4,4'-Diphenylmethandiisocyanat, 1 - 12 Gew. % an 2,4'-Diphenylmethandiisocyanat und maximal 2 Gew. % an 2,2'-Diphenylmethandiisocyanat sowie 35 - 65 Gew. % an Tri- und höherfunktionellen Polyisocyanaten, bevorzugt 35 -55 Gew. % an 4,4'-Diphenylmethandiisocyanat, 2 - 10 Gew. % an 2,4'-Diphenylmethandiisocyanat und maximal 1 Gew. % an 2,2'-Diphenylmethandiisocyanat sowie 40 - 60 Gew. % an Tri- und höherfunktionellen Polyisocyanaten, bezogen auf das Gewicht der zweiten Fraktion.

Dies erlaubt auch, die bei der Destillation und / oder Kristallisation anfallenden Nebenströme zurückzuführen und auf die Ausschleusung von im Wesentlichen monomeren (d.h. Diisocyanat enthaltenden), in größeren Mengen 2,4'-MDI enthaltenden Strömen zu verzichten. Das 2,4'-MDI dient statt dessen überwiegend als Bestandteil der zweiten Fraktion und wird mit der zweiten Fraktion ausgeschleust, so dass das 2,4'-MDI nicht technisch aufwendig isoliert, transportiert und /oder einer anderen Verwendung zugeführt werden muss.

Die nachfolgenden Beispiele sollen die Erfindung näher erläutern, ohne sie jedoch in ihrem Umfang zu begrenzen.

### Beispiele

### Beispiel 1

### a) Herstellung eines Gemisches von Di- und Polyaminen:

In einem Rührgefäß werden 2600 g Anilin bei 25°C mit 1000 g Formalin (30 Gew. %ige wässrige Lösung von Formaldehyd, bezogen auf das Gewicht der Lösung) unter Rühren intensiv vermischt, wobei sich die Mischung auf 60°C erwärmt. Man stellt den Rührer ab und trennt die sich oben abscheidende Wasserphase ab. Anschließend mischt man unter erneutem Rühren und Kühlen 680 g 30 Gew. %ige wässrige Salzsäure zu, wobei eine Temperatur von 45°C gehalten wird. Nach 15 min weiterem Rühren bei dieser Temperatur wird die Kühlung durch Heizung ersetzt und die Mischung im Lauf von 120 min unter 5 bar Druck gleichmäßig auf 140°C aufgeheizt und danach 15 min bei dieser Temperatur gehalten.

Anschließend wird die Mischung auf 100°C abgekühlt, auf Normaldruck entspannt und durch Zugabe von 540 g 50 Gew. %ige wässrige Natronlauge unter Rühren neutralisiert. Nach Abstellen des Rührers lässt man die Phasen absitzen und saugt die untere Wasserphase ab. Anschließend destilliert man zunächst unter Normaldruck überschüssiges Anilin mit verbliebenem Restwasser ab und entfernt die Anilinreste durch Andestillieren des erhaltenen Polyamingemischs bei 100 mbar und 250°C.

Man erhält 1900 g eines Gemisches von Di- und Polyaminen der folgenden Zusammensetzung:
4,4'-MDA: 60,1 Gew. %,
2,4'-MDA: 6,0 Gew. %,
2,2'-MDA: 0,2 Gew. %,
höhermolekulare Polyamine: 33,7 Gew. %, bezogen auf das Gewicht des Gemisches.

### b) Herstellung eines Gemisches von Di- und Polyisocyanaten:

Man löst in einem weiteren Rührreaktor die 1900 g des in Beispiel 1 a) erhaltenen Gemisches von Di- und Polyaminen in 5700 g Chlorbenzol auf. In einem zweiten Gefäß stellt man durch Lösen von 3800 g Phosgen in 7600 g Chlorbenzol unter Kühlen auf 0°C eine 33 Gew.%ige (bezogen auf das Gewicht der Lösung) Phosgenlösung her und vermischt unter intensivem Rühren die Amin- und Phosgenlösungen. Die entstandene Feststoffsuspension wird nun langsam aufgeheizt, wobei Chlorwasserstoffgas entsteht, das in geeigneter Weise abgeleitet wird. Dabei entsteht eine homogene Lösung des Polyisocyanats. Durch Destillation wird nun das Lösungsmittel abgetrennt, wodurch man 2370 g eines Gemisches von Di- und Polyisocyanaten der folgenden Zusammensetzung erhält:
4,4'-MDI: 59,3 Gew. %,
2,4'-MDI: 5,5 Gew. %,
2,2'-MDI: 0,2 Gew. %,
höhermolekulare Polyisocyanate: 35 Gew. %, bezogen auf das Gewicht des Gemisches.

### c) Herstellung der ersten Fraktion (technisch reines von 4,4'-MDI):

In einer Blasendestillation wird das in Beispiel 1 b) erhaltene Gemisch von Di- und Polyisocyanaten bei 10 mbar Druck und 215°C Sumpftemperatur soweit andestilliert, dass 950 g Destillat (Fraktion enthaltend mindestens 95 Gew.-% Diisocyanate) erhalten werden, wobei 1420 g eines Sumpfprodukts mit 400 mPas Viskosität verbleiben.

Das Destillat (Fraktion enthaltend mindestens 95 Gew.-% Diisocyanate) wird in einer Trennwandkolonne entsprechend EP-A-1475367 weiter aufgearbeitet. Dabei werden 59 g/ h des Destillats aus der Blasendestillation der Trennwandkolonne im Bereich der Trennwand zugeführt. Der Trennwandkolonne wird ein Sumpfstrom von 51 g/ h mit einem Gehalt an 4,4'-MDI von 98 Gew. % und einem Gehalt an 2,4'-MDI von 2 Gew.-% entnommen und als erste Fraktion ausgeschleust. Ferner wird ein Kopfstrom von 0,7 g/ h mit einer Zusammensetzung 25 Gew. % 2,2'-MDI, 73 Gew. % 2,4'-MDI und 2 Gew.-% 4,4'-MDI sowie ein Seitenstrom von 6,1 g/ h mit einer Zusammensetzung von 42,6 Gew. % 2,4'-MDI und 57,4 % 4,4'-MDI entnommen.

Als Stoffaustauschelemente werden in der Trennwandkolonne Gewebepackungen mit 500 m²/ m³ spezifischer Oberfläche eingesetzt. Die Rektifizierzone und die Abtriebszone besitzen jeweils 8 Trennstufen, die Vorfraktionierungszone und die Hauptfraktionierungszone jeweils 12 Trennstufen oben und unten, d.h. oberhalb und unterhalb der Zuführung des Feedstroms in die Vorfraktionierungszone bzw. der Seitenstromentnahme aus der Hauptfraktionierungszone. Der Kopfdruck beträgt 6 mbar. Der Rücklauf an der Destillatentnahme beträgt 90:1, an der Seitenstromentnahme 2,6:1.

### d) Herstellung der zweiten Fraktion (polymeres MDI):

Die in Beispiel 1 c) erhaltenen Nebenströme, d.h. der Kopfstrom (11,5 g) sowie der Seitenstrom (98,5 g) werden mit dem in Beispiel 1 c) ebenso erhaltenen Sumpfstrom (1420 g) abgemischt, wobei 1530 g polymeres MDI als zweite Fraktion erhalten werden.

Insgesamt werden also 822 g technisch reines 4,4'-MDI als erste Fraktion und 1530 g polymeres MDI mit 200 mPas Viskosität als zweite Fraktion erhalten.

### Beispiel 2

### a) Herstellung eines Gemisches von Di- und Polyaminen:

In einem Rührgefäß werden 1800 g Anilin bei 30°C mit 1000 g Formalin (30 Gew. %ige wässrige Lösung von Formaldehyd, bezogen auf das Gewicht der Lösung) unter Rühren intensiv vermischt, wobei sich die Mischung auf 80°C erwärmt. Man stellt den Rührer ab und trennt die sich oben abscheidende Wasserphase ab. Anschließend mischt man unter erneutem Rühren und Kühlen 230 g 30 Gew. %ige wässrige Salzsäure zu, wobei eine Temperatur von 45°C gehalten wird. Nach 15 min weiterem Rühren bei dieser Temperatur wird die Kühlung durch Heizung ersetzt und die Mischung im Lauf von 150 min unter 5 bar Druck gleichmäßig auf 140°C aufgeheizt und danach 20 min bei dieser Temperatur gehalten.

Anschließend wird die Mischung auf 100°C abgekühlt, auf Normaldruck entspannt und durch Zugabe von 180 g 50 Gew. %ige wässrige Natronlauge unter Rühren neutralisiert. Nach Abstellen des Rührers lässt man die Phasen absitzen und saugt die untere Wasserphase ab. Anschließend destilliert man zunächst unter Normaldruck überschüssiges Anilin mit verbliebenem Restwasser ab und entfernt die Anilinreste durch Andestillieren des erhaltenen Polyamingemischs bei 100 mbar und 250°C.

Man erhält 1880 g eines Gemisches von Di- und Polyaminen der folgenden Zusammensetzung:
4,4'-MDA: 44,5 Gew. %,
2,4'-MDA: 7,3 Gew. %,
2,2'-MDA: 0,5 Gew. %,
höhermolekulare Polyamine: 47,7 Gew. %, bezogen auf das Gewicht des Gemisches.

### b) Herstellung des Polyisocyanatgemischs:

Das in Beispiel 2 a) erhaltenen Gemisch von Di- und Polyaminen wird in analoger Weise wie in Beispiel 1 beschrieben mit Phosgen in Chlorbenzol mit dem gleichen molaren Verhältnis von Phosgen zu Aminogruppen wie in Beispiel 1 zum Polyisocyanatgemisch umgesetzt.

Man erhält 2330 g eines Polisocyanatgemischs der folgenden Zusammensetzung:
4,4'-MDI: 44,1 Gew. %,
2,4'-MDI: 7,2 Gew. %,
2,2'-MDI: 0,5 Gew. %,
höhermolekulare Polyisocyanate: 48,2 Gew. %, , bezogen auf das Gewicht des Gemisches.

### c) Herstellung der ersten Fraktion (technisch reines von 4,4'-MDI):

In einer Blasendestillation wird das in Beispiel 2 b) erhaltene Gemisch von Di- und Polyisocyanaten bei 10 mbar Druck und 215°C Sumpftemperatur soweit andestilliert, dass 280 g Destillat (Fraktion enthaltend mindestens 95 Gew.-% Diisocyanate) erhalten werden, wobei 2050 g Sumpfprodukt verbleiben.

Das Destillat (Fraktion enthaltend mindestens 95 Gew.-% Diisocyanate) wird in einer Trennwandkolonne entsprechend EP-A-1475367 analog zu Beispiel 1 weiter aufgearbeitet. Dabei werden 59 g/ h des Destillats aus der Blasendestillation der Trennwandkolonne im Bereich der Trennwand zugeführt. Der Trennwandkolonne wird ein Sumpfstrom von 46,9 g/ h mit einem Gehalt an 4,4'-MDI von 98 Gew. % und einem Gehalt an 2,4'-MDI von 2 Gew.-% entnommen und als erste Fraktion ausgeschleust. Ferner wird ein Kopfstrom von 1,2 g/ h mit einer Zusammensetzung 25 Gew. % 2,2'-MDI, 73 Gew. % 2,4'-MDI und 2 Gew.-% 4,4'-MDI sowie ein Seitenstrom von 10,7 g/ h mit einer Zusammensetzung enthaltend 54,9 Gew. % 2,4'-MDI und 45,1 Gew.-% 4,4'-MDI entnommen.

### d) Herstellung der zweiten Fraktion (polymeres MDI):

Die in Beispiel 2 c) erhaltenen Nebenströme, d.h. der Kopfstrom (6 g) sowie der Seitenstrom (51 g) werden mit dem in Beispiel 2 c) ebenso erhaltenen Sumpfstrom (2050 g) und einem weiteren Gemisch von Di- und Polyisocyanaten (2330 g), das gemäß Beispiel 2 b) hergestellt wurde, gemischt. Aus dem so erhalten Gemisch werden wiederum in einer Blasendestillation bei 10 mbar Druck und 215°C Sumpftemperatur 280 g Destillat abdestilliert, wobei 4157 g Sumpfprodukt mit 200 mPas Viskosität verbleiben. Von diesem Sumpfprodukt werden 2107 g als Fertigprodukt (zweite Fraktion) entnommen und 2050 g wieder zur Abmischung mit den in Beispiel 2 c) erhaltenen Kopf- und Seitenströmen rückgeführt. Anschließend wird das Destillat analog zu Beispiel 2 c) in der Destillation in der Trennwandkolonne weiterverarbeitet.

Insgesamt werden bei wiederholter Recyclisierung von Sumpfprodukt und Destillat im Gleichgewicht 223 g technisch reines 4,4'-MDI als erste Fraktion und 2107 g polymeres MDI mit 200 mPas Viskosität als zweite Fraktion erhalten.

## Patentansprüche

1. Verfahren zur Herstellung von 4,4'-Diphenylmethandiisocyanat, bei dem
a) Anilin und Formaldehyd im Molverhältnis 1,7 bis 4 : 1 in Gegenwart eines sauren Katalysators zu Di- und Polyaminen der Diphenylmethanreihe umgesetzt werden, und
b) die Di- und Polyamine mit Phosgen zu den entsprechenden Di- und Polyisocyanaten der Diphenylmethanreihe umgesetzt werden, und ggf. destillativ getrennt werden, wobei ein Gemisch von Di- und Polyisocyanaten enthaltend 44 - 80 Gew.-% 4,4'-Diphenylmethandiisocyanat, sowie 1 bis 12 Gew.-% an 2,4'- und / oder 2,2'-Diphenylmethandiisocyanat in der Summe, und 10 bis 55 Gew.-% an Tri- und höherfunktionellen Polyisocyanaten, bezogen auf das Gewicht des Gemisches von Di- und Polyisocyanaten, erhalten wird, und
c) das Gemisch von Di- und Polyisocyanaten durch Destillation und / oder durch Kristallisation in genau zwei Fraktionen aufgetrennt wird, wobei die erste Fraktion in Mengen von 5 - 40 Gew.-% der Menge des Gemisches der Di- und Polyisocyanate erhalten wird und die zweite Fraktion in Mengen von 60 - 95 Gew.-% der Menge des Gemisches der Di- und Polyisocyanate erhalten wird, und wobei die erste Fraktion mindestens 97 Gew.-% an 4,4'-Diphenylmethandiisocyanat sowie maximal 3 Gew. % an 2,4'-Diphenylmethandiisocyanat, bezogen auf das Gewicht der ersten Fraktion, enthält und die zweite Fraktion 30 - 60 Gew. % an 4,4'-Diphenylmethandiisocyanat, 1 - 12 Gew. % an 2,4'-Diphenylmethandiisocyanat und maximal 2 Gew. % an 2,2'-Diphenylmethandiisocyanat sowie 35 - 65 Gew. % an Tri- und höherfunktionellen Polyisocyanaten, bezogen auf das Gewicht der zweiten Fraktion, enthält.

2. Verfahren nach Anspruch 1, bei dem die Destillation und / oder Kristallisation mehrere Destillations- und / oder Kristallisationsschritte enthält.

3. Verfahren nach Anspruch 1, bei dem in Schritt c) zunächst aus dem Gemisch von Di- und Polyisocyanaten durch Destillation und / oder durch Kristallisation eine Fraktion enthaltend mindestens 95 Gew.-% Diisocyanate, bezogen auf das Gewicht der Fraktion, abgetrennt wird, wobei ein Sumpfstrom enthaltend Di- und Polyisocyanate erhalten wird.

4. Verfahren nach Anspruch 3, bei dem die abgetrennte Fraktion enthaltend mindestens 95 Gew.-% Diisocyanate mindestens 97 Gew.-% an 4,4'-Diphenylmethandiisocyanat sowie maximal 3 Gew. % an 2,4'-Diphenylmethandiisocyanat, bezogen auf das Gewicht der abgetrennten Fraktion enthaltend mindestens 95 Gew.-% Diisocyanate, enthält.

5. Verfahren nach Anspruch 3, bei dem die abgetrennte Fraktion enthaltend mindestens 95 Gew.-% Diisocyanate in weiteren Destillations- und / oder Kristallisationsschritten von den 2,2'- und 2,4' -Isomeren sowie anderen Verunreinigungen zumindest teilweise befreit wird, wobei 2,2'-MDI, 2,4'-MDI und / oder andere Verunreinigungen enthaltende Nebenströme anfallen.

6. Verfahren nach Anspruch 5, bei dem der Sumpfstrom enthaltend Di- und Polyisocyanate und die 2,2'-MDI, 2,4'-MDI und / oder andere Verunreinigungen enthaltenden Nebenströme vereinigt werden.

7. Verfahren nach Anspruch 5, bei dem der Sumpfstrom enthaltend Di- und Polyisocyanate und die 2,2'-MDI, 2,4'-MDI und / oder andere Verunreinigungen enthaltenden Nebenströme vermischt werden und das Gemisch erhitzt und anschließend destillativ aufgetrennt wird, wobei als Sumpfstrom die zweite Fraktion erhalten wird.

8. Verfahren nach Anspruch 5, bei dem der Sumpfstrom enthaltend Di- und Polyisocyanate und die 2,2'-MDI, 2,4'-MDI und / oder andere Verunreinigungen enthaltenden Nebenströme vereinigt werden und dem in Schritt b) erhalten Gemisch an Di- und Polyisocyanaten zugemischt werden und das resultierende Gemisch destilliert wird , wobei die zweite Fraktion als Sumpfstrom erhalten wird.
